# EUROPEAN PATENT SPECIFICATION

(11) **EP 0 873 731 B1**
(45) Date of publication and mention of the grant of the patent: **12.04.2000**
(21) Application number: 98303114.7
(22) Date of filing: 22.04.1998
(51) Int. Cl.: A61F 2/06

(54) **Stent-crimping tool and method of use**
Werkzeug zum Komprimieren eines Stents und Verfahren zum Gebrauch
Outil pour comprimer un stent et procédé d'utilisation

(30) Priority: 22.04.1997 US 837771
(43) Date of publication of application: 28.10.1998
(73) Proprietor: Advanced Cardiovascular Systems, Inc., Santa Clara, CA 95054-8167 (US)
(72) Inventor: Morales, Stephen A., Mountain View, California 94041 (US)
(74) Representative: McLeish, Nicholas Alistair Maxwell

(56) References cited:
- EP-A- 0 630 623
- EP-A- 0 826 346
- US-A- 5 195 539
- US-A- 5 672 169

## Description

This invention relates to a stent-crimping device and method of using the device that enables an end user to firmly crimp a stent onto the distal end of a catheter assembly.

In a typical percutaneous transluminal coronary angioplasty (PTCA) procedure for compressing lesion plaque against the artery wall to dilate the arterial lumen, a guiding catheter is introduced percutaneously into the cardiovascular system of a patient through the brachial or femoral arteries and is advanced through the vasculature until the distal end is in the ostium. A guide wire and a dilatation catheter having a balloon on the distal end are introduced through the guiding catheter with the guide wire sliding within the dilatation catheter. The guide wire first is advanced out of the guiding catheter into the coronary vasculature of the patient, and the dilatation catheter is advanced over the previously advanced guide wire until the dilatation balloon is properly positioned across the lesion. Once in position across the lesion, a flexible, expandable, pre-formed balloon is inflated to a pre-determined size at relatively high pressures to radially compress the atherosclerotic plaque of the lesion against the inside of the artery wall and to thereby dilate the lumen of the artery. The balloon then is deflated to a small profile, so that the dilatation catheter can be withdrawn from the vasculature of the patient and blood flow resumed through the dilated artery. While this procedure is typical, it is not the only method used to accomplish an angioplasty procedure. Further, other methods are well known to open a stenosed artery, such as atherectomy procedures, plaque-dissolving drugs, and the like.

In angioplasty procedures of the kind referenced above, restenosis of the artery may occur, and a further angioplasty procedure, a surgical bypass operation, or some method of repairing or strengthening the area may be required to treat the restenosis. To reduce the chance of restenosis and to strengthen the area, a physician can implant an intravascular prosthesis for maintaining vascular patency, such prosthesis typically being referred to as a stent. A stent is a device used to hold tissue in place, or to provide support for a graft or for tissue joined while healing is taking place. A variety of devices are known in the art for use as stents, including coiled wires and wire mesh sleeves, in a variety of patterns, that can be crimped onto a balloon catheter, and then expanded after being positioned intraluminally on the balloon catheter, and which have the capacity to retain the expanded form. Typically, the stent is mounted and crimped onto the balloon portion of the catheter and then advanced to a location inside the artery at the lesion. The stent then is expanded to a larger diameter by the balloon portion of the catheter, in order to implant the stent in the artery at the lesion. Examples of stents and delivery catheters of the type described herein are disclosed in more detail in U.S. Patent No. 5,102,417 (Palmaz); U.S. Patent No. 5,514,154 (Lau et al.); and U.S. Patent No. 5,569,295 (Lam).

If the stent is not tightly crimped onto the catheter balloon portion, however, when the catheter is advanced in the vasculature, the stent may slide off the catheter balloon portion in the coronary artery prior to expansion, and thereby may block the flow of blood, necessitating procedures to remove the stent.

In procedures where the stent is placed over the balloon portion of the catheter for delivery, the stent first must be crimped onto the balloon portion to prevent the stent from sliding off the catheter when the catheter is advanced in the vasculature of the patient. In the past, the crimping procedure often was done by hand, which tended to result in uneven force being applied, such that the stent was not crimped onto the balloon uniformly. In addition, hand crimping makes it difficult to determine when a uniform and reliable crimp has been applied or whether the stent has damaged the balloon as a result of the crimping process. Though some tools, such as ordinary pliers, have been used to crimp a stent onto a balloon, these tools have not been entirely adequate in achieving a uniform crimp.

Some specialist crimping tools have been designed. For example in EP-A-0,630,623 there is described a stent-loading mechanism for automatically loading a stent onto a balloon delivery catheter. The device comprises a pair of plates having substantially flat and parallel surfaces that move in a rectilinear fashion with respect to each other. A stent-carrying catheter can be disposed between these surfaces to affix the stent onto the outside of the catheter by providing relative motion between the plates. The plates may have multiple degrees of freedom and force-indicating transducers to measure and indicate the force applied to the catheter during affixation of the stent.

Another embodiment of the stent-loading mechanism comprises a tubular member housing an elongated elastic bladder that surrounds the stent to be loaded. The distal end of the balloon catheter assembly and the stent are placed inside the tubular member and pressurized fluid is applied to the bladder to compress and affix the stent onto the outside of the catheter assembly.

Crimping tools are also known in other fields. US-A-5,195,539 describes a device for compressing slow recovery earplugs. The device comprises a flexible strip having an elongate compression portion having first and second ends and a base portion at the first end of the compression portion. The base portion has a width greater than that of the compression portion. An opening is provided proximate the compression portion having a width transverse of the compression portion larger than the width of the compression portion. The compression portion is at least 15mm wide and of sufficient length that the second end can pass through the opening to form a tubular compression means having a diameter at least that of a slow recovery earplug.

Nevertheless, there remains a need for improved tools and methods to secure stents onto the balloon portions of catheters.

This invention is directed to a vascular prosthesis crimping device or tool which enables a stent to be crimped onto the balloon portion of a catheter in a substantially uniform and tight manner, to better secure the stent onto the catheter for delivery of the stent through the vasculature of a patient, while at the same time permitting uniform expansion of the stent in an artery or a vein, duct, or other vessel or lumen. The present invention solves several deficiencies that have been experienced with prior art methods of crimping stents onto the balloons of balloon catheters.

According to a first aspect of the present invention there is provided a stent-crimping tool having the features set out in claim 1.

In an exemplary embodiment of the present invention, the stent-crimping tool includes a radially compressible and resiliently and radially expandable cylindrical loop portion having opposed side edges extending from the loop portion. These side edges are secured to pivoting arm portions of the device. A user can inwardly and radially compress the inner diameter of the loop portion by moving the pivoting arm portions downwardly to substantially uniformly and tightly crimp the stent onto the balloon catheter assembly which is inserted within the loop portion. The loop portion is returned to its expanded state by bringing the pivoting arm portions back to the un-pivoted position, thereby allowing the crimped stent-and-balloon-catheter assembly to be withdrawn by the user.

The stent-crimping tool enables the stent to be crimped onto the distal end of a balloon catheter substantially uniformly and tightly, reducing the risk that the stent may shift or slide off the balloon portion of the catheter during delivery. The tool is easy to use and is particularly well suited for use in compressing stents onto balloon catheters of different manufacturers, due to its simplicity, low manufacturing and assembly cost, an adaptability to compressing stents of different length and diameter. For the same reasons, the tool is ideally suited as a one-time use disposable crimping tool, thereby eliminating the need for sterilization of the device between uses.

These and other advantages of the invention will become apparent from the following detailed description, when taken in conjunction with the accompanying drawings.

### BRIEF DESCRIPTION OF THE DRAWINGS

FIGURE 1 is a perspective view of an exemplary embodiment of the present invention, in its open position, in which the loop portion of the device is fully expanded for receipt of a stent to be compressed onto a balloon catheter.

FIG. 2 is a top plan view of the main body portion of the embodiment illustrated in FIG. 1.

FIG. 3 is a cross-sectional view through the view lines 3-3 of FIG 2.

FIG. 4 is a perspective side view of the loop portion of a stent-crimping tool according to the invention in the threaded orientation.

FIG. 5 is a plan view of the sheet of material forming the loop portion of the stent-crimping tool of FIG. 4 prior to being threaded.

FIG. 6 is a bottom view of the clips of a stent-crimping tool according to the invention which are used to secure the loop portion to the main body portion.

FIG 7 is a side view of the securing clips as illustrated in FIG. 6.

FIG. 8 is a side view of a stent-crimping tool according to the invention packaged to maintain its loop portion, in its open position, wherein the loop portion cannot be radially compressed.

FIG. 9 is an exploded side view of the parts of a stent-crimping tool according to the invention, prior to final assembly, and the packaging thereof.

FIG. 10 is a side view of a stent-crimping tool according to the invention, with packaging removed, illustrating the tool in the open position prior to radial compression of the loop portion.

FIG. 11 is a side view of the stent-crimping tool of FIG. 10, with the arm portions of the main body portion pivoted downwardly away from the intermediate portion so as to cause the loop portion to constrict in diameter and to thereby crimp a stent around the balloon portion of the catheter which has been positioned within the loop portion.

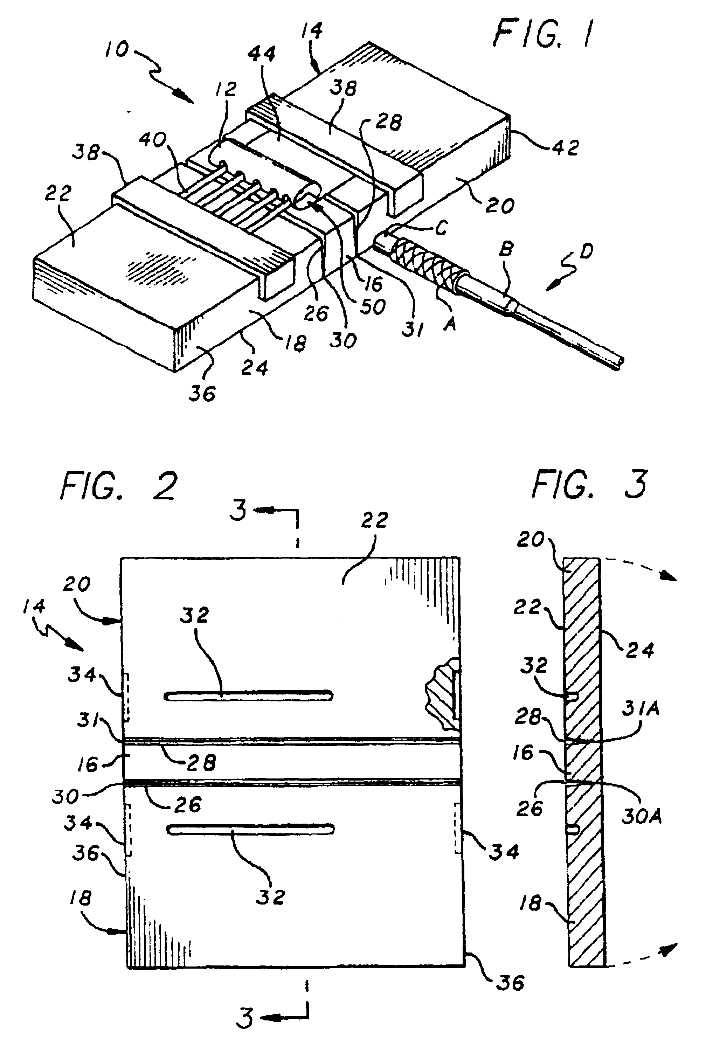

The invention comprises a tool 10 and method of using a tool that is useful in uniformly and tightly crimping an intravascular stent A onto the collapsed balloon portion B of a balloon catheter assembly D, which balloon is adjacent the distal end C of the catheter assembly. In the exemplary embodiment as illustrated in FIGS. 1 and 10, the tool 10 is adapted to be held in the hand of the user. The user will insert the stent A, which previously has been placed over the collapsed balloon B, into the loop portion 12 of the tool 10 to enable the stent A and the catheter D to be supported in the tool, and to enable the user to apply compressive force to the tool to substantially uniformly and tightly crimp the stent A onto the collapsed balloon B of the catheter D.

The loop portion 12 of the tool 10 is affixed to a base portion 14. The circumference of the loop portion 12 is variable. The base portion 14 has an intermediate portion 16 which is pivotally connected to two pivoting arm portions 18 and 20. The base portion 14 has a top surface 22 on which the loop portion 12 rests. The base portion 14 ideally comprises a single flat piece of material, such as plastic, with a pair of parallel, longitudinal, V-shaped slits 26 and 28 extending from the top surface 22 almost all the way to the bottom surface 24, the slits 26 and 28 each terminating in a lower parallel edge 30, 31, such that each slit forms a hinge 30A, 31A to permit the pivoting arm portions 18 and 20 to swing downwardly away from the intermediate portion 16. When the base portion 14 is formed from a single piece of plastic (or metal), then the hinges 30A and 30B can be said to be living hinges formed in base portion 14. Alternatively, the base portion 14 and the pivoting arm portions 18,20 can be formed separately and then hinges of conventional design can be added to connect the pivoting arm portions to the base. Referring to FIGS. 2 and 3, pivoting arm portions 18 and 20 each have a first engagement means, for example slots 32, formed in the top surface 22 of the base portion 14. A recess 34 is formed on one or more of the side edges 36 of each pivoting arm 18, 20, as is best shown in FIG. 2. A clip portion 38 is used to secure the loop portion 12 to the base portion 10 on each pivoting arm.

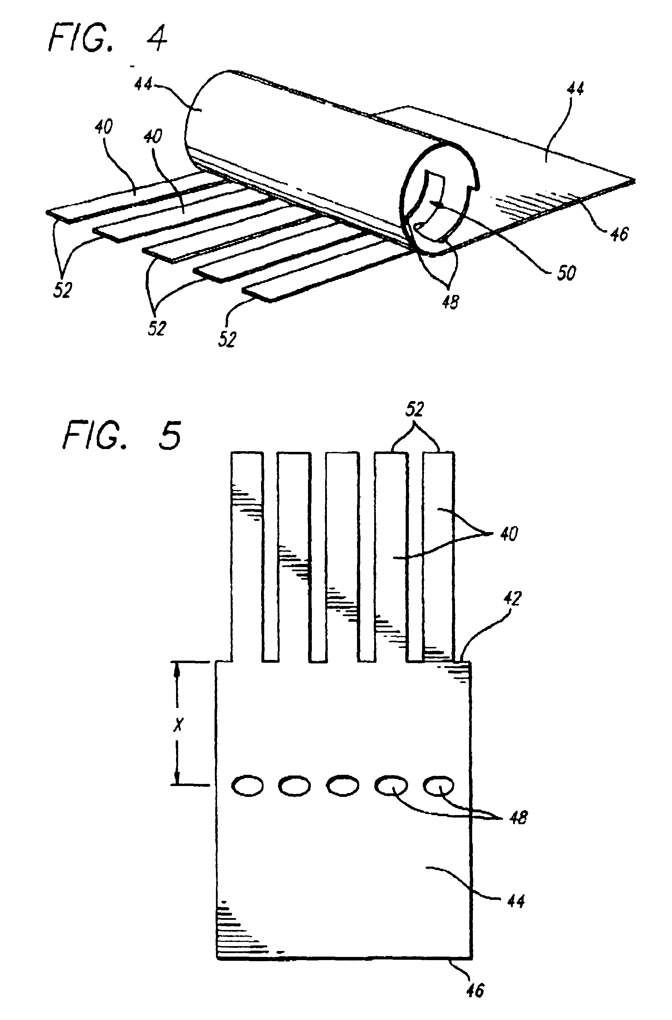

Referring to FIGS. 4 and 5, a preferred embodiment of loop portion 12 is shown in greater detail. The loop portion 12 comprises a sheet 44 of thin and flexible yet strong material, such as the polyester film sold under the tradename "MYLAR" by E.I. duPont deNemours and Company, and has a plurality of elongate straps 40 extending from a first end 42 of the sheet 44 and extending opposite a second end 46 of the sheet 44. Other known flexible materials can be used. Apertures 48 formed in the sheet 44 are adapted to receive the straps 40 when threaded therethrough, as shown in FIGS. 1 and 4, thereby forming a generally cylindrical opening 50 for receiving a stent A after it has been placed on the distal end C of the balloon catheter assembly B. Each strap 40 has a terminal end region 52. By pulling the terminal end regions 52 of the straps 40 and the second end 46 of the sheet 44 away from each other, the generally cylindrical opening 50 will constrict in size, from its larger size shown in FIGS. 1, 4, 8 and 9, to the constricted size shown in FIG. 10.

As seen in FIG. 5, the distance X between the aperture 48 and the first end 42 of the sheet 44 will determine the smallest possible diameter of the loop portion 12. Those skilled in the art will appreciate that the distance X can be varied, depending upon the diameter of the stent and of the balloon, and ultimately upon the diameter of the stent on the balloon after crimping. The thickness of the polyester film forming the loop portion 12 is an important consideration as to the capacity of the loop to rebound open after the stent is crimped. Thus, the polymer film preferably has a thickness in the range of 0.05 to 0.20mm (0.002 to 0.008 inches). With this range of thickness for the polymer film, it is flexible and durable, and will readily rebound open to return the loop portion 12 approximately to its starting diameter after the crimping procedure has been accomplished and the stent-and-balloon-catheter assembly have been removed from the tool.

In a preferred embodiment of the invention, the straps 40 can be about 5 mm wide (0.2 inches), with about 2.5 mm (0.1 inches) of open space between each strap and an adjacent strap. The entire length of the sheet 44, the length of the straps 40 and the spacing of the apertures 48 from the first end 42, can be chosen depending on the size of the loop portion 12 desired. In lieu of a sheet of polyester film or other sheet material, a plurality of wires or cords can be utilized to form the loop portion 12.

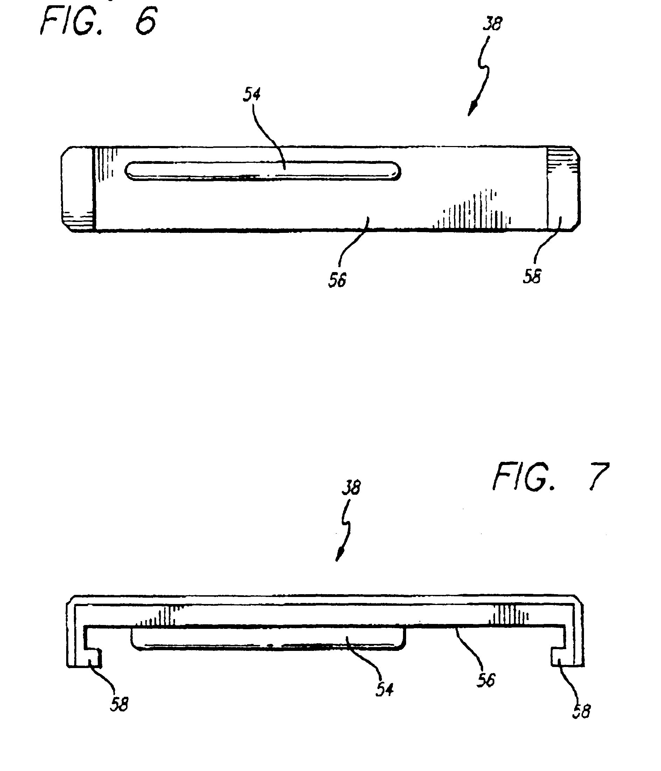

The end regions 52 of the elongate straps and the second end of the sheet 44 forming the loop portion 12 are secured to the tool 10 for radially compressible and expandable movement as follows. Referring to FIGS. 2, 3, 6 and 7, the engagement means or slots 32 formed on the top surface 22 of the pivoting arm portions 18 and 20 (FIGS. 2 and 3), are adapted to receive a complementary engagement portion, for example, the bar portion 54 which extends from a bottom surface 56 of the clip portion 38. The clip ends 58 are formed on the ends of the clip portions 38 (FIGS. 6 and 7). After placing the loop portion 12 on the top surface 22 of the intermediate portion 16 and extending the terminal end regions 52 of the elongate straps 40 and second end 46 of the sheet 44 over the slots 32 in the pivoting arms 18, 20, the clip portions 38 are snapped onto the pivoting arms 18, 29 the complementary bar portions 54 fitting into the appropriate slots 32 and clip ends 58 fitting into recesses 34. This secures the terminal end regions 52 of the elongate straps 40 and the second end 46 of the sheet 44 comprising the loop portion 12, yet allows the portion of loop portion defining the generally cylindrical opening 50 to change configuration unencumbered by riding on the intermediate portion 16. In addition to this mechanical affixation, adhesives and other means can be utilized to secure the elongate straps 40 and the end regions 52 thereof to the base portion 14. An advantage of stent-crimping tool the described is that the components comprising it, e.g., the base portion 14, the loop portion 12 and the clip portions 38 are all relatively low in cost, and the base portion 14 and the clip portions 38 will accommodate a loop portion 12 of the desired diameter and length, to accept a balloon catheter D and a stent A of desired diameter and length.

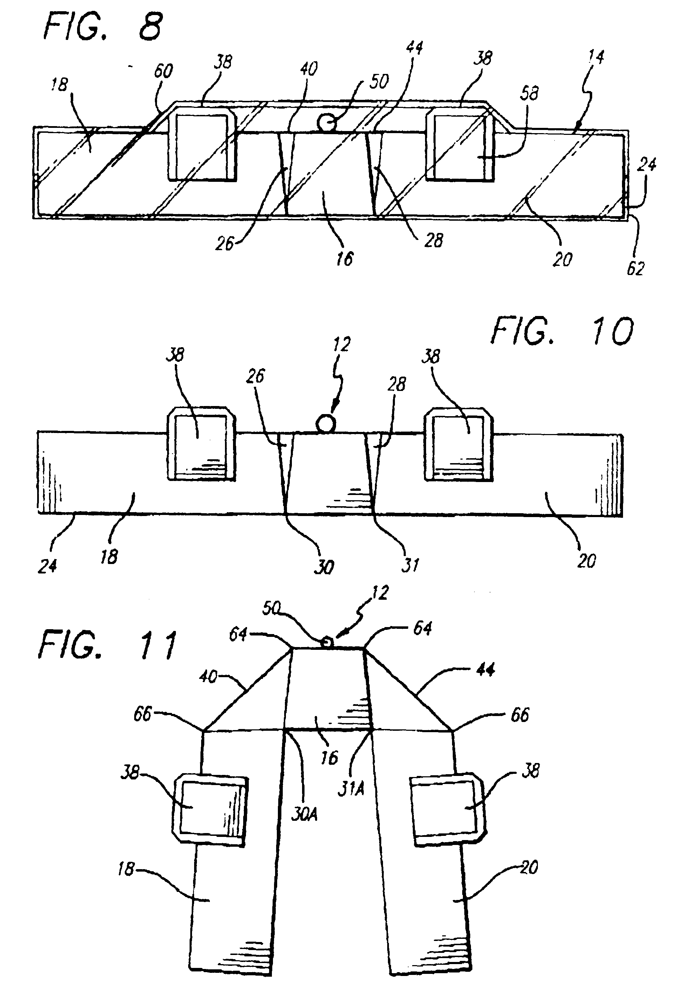

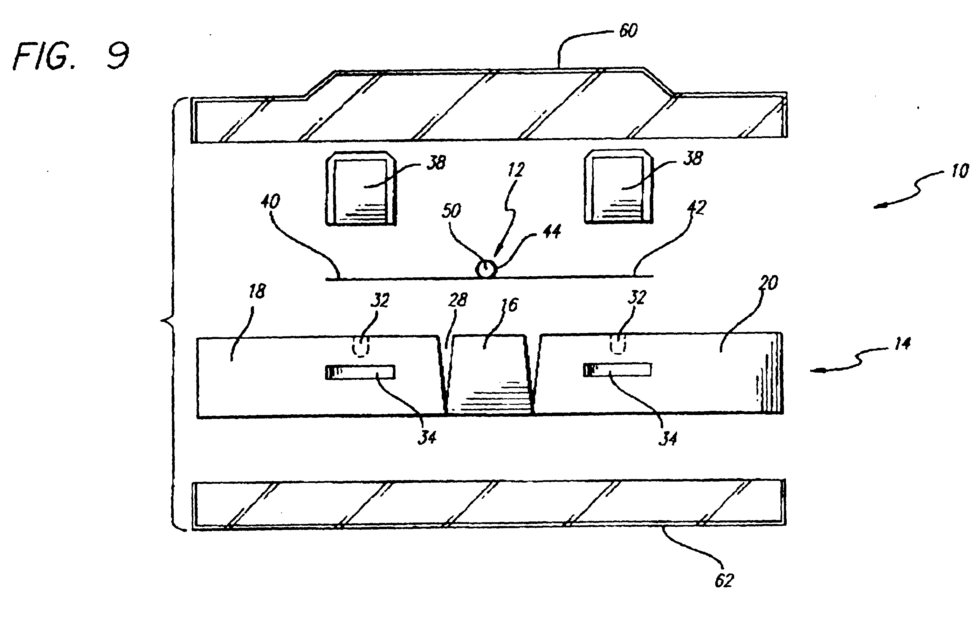

Referring to FIG. 8, the top surface 22 of the base portion 14, the clip portions 38, and the loop portion 12 are covered with a protective layer 60 which cushions the top surface 22 and the loop portion 12, and prevents the loop portion 12 from being inadvertently crushed. The protective layer 60 can comprise bubble pack material, a vacuum-formed plastic cover, or other known materials. To prevent the pivoting arms 18, 20 and the intermediate portion 16 of the base portion 14 from being pivoted, a rigid support sheet 62 preferably is positioned on the bottom surface 24 of the base portion 14. Other means can be provided to protect the stent-crimping tool 10, such as a case or box to surround the device in a sterile environment. FIG. 9 is an exploded side view of the stent-crimping tool 10 and its packaging. FIG. 10 shows the stent-crimping tool 10 after it has been removed from the packaging (including the protective layer 60 and the rigid support sheet 62) and ready for use.

Referring to FIGS 1 and 11, in a preferred method of operation, a user will load a stent A onto a deflated (unused) balloon portion B of a balloon catheter assembly D. The balloon catheter assembly then is inserted into stent A, such that stent A overlies the balloon portion B. To enable the stent A to be crimped onto the catheter balloon portion B, the stent A and the balloon portion B are inserted within loop the portion 12 and supported in the middle of the loop portion 12 which is carried on the intermediate portion 16 of the base portion 14. At this point, the stent A is not fixed onto catheter assembly D, because a stent A has not been compressed.

To crimp stent A onto the catheter balloon portion B, the user of the stent-crimping tool 10 simultaneously swings pivoting arm portions 18, 20 downwardly together relative to the intermediate portion 16. Pivoting arm portions 18, 20 downwardly causes the terminal ends 52 of the elongate straps 40 and the second end 46 of the sheet 44 to be pulled in opposite directions in a noose-like manner. The elongate straps 40 and the sheet portion 44 on the sides of the loop portion 12 will extend between opposite edges 64 and 66 of intermediate portion 16 and pivoting arm portions (18, 20), respectively. As swinging arm portions 18, 20 are swung down from an intermediate portion 16, the generally cylindrical opening 50 in the loop portion 12 will constrict to a smaller inner diameter, compressing a stent A radially inwardly and tightly onto the balloon portion B at a substantially uniform rate.

If further crimping of the stent A onto catheter the balloon portion B is desired, the user may rotate the crimped stent A and the catheter balloon portion B and/or the stent and the catheter balloon portion forward or backward in the loop portion 12, and repeat the crimping procedure until the stent A is as tightly crimped onto catheter balloon portion B as desired.

After the stent A has been crimped onto the catheter balloon portion B, the user will swing pivoting arm portions 18, 20 back up, thereby enlarging the generally cylindrical opening 50 and permitting the balloon catheter assembly with the crimped stent thereon to be removed from the generally cylindrical opening 50. The balloon catheter assembly D, with the stent A crimped thereon, then may be inserted into the body of the patient for deployment of the stent A (deployment is not illustrated in the drawing figures).

As will be appreciated by those skilled in the art, the stent-crimping tool of the present invention is designed both for single use applications in a catheterization lab, or for multiple use applications in a sterile environment in a high-volume manufacturing facility. In the manufacturing environment, where sterile conditions exist, the stent-crimping tool can be used to repeatedly crimp stents onto balloons until the polyester film wears out and has to be replaced. Thus, repeated uses are contemplated for controlled sterile environments, however, single use applications are required when used by catheterization lab physicians or other medical personnel.

While in the preferred embodiments the stent referenced herein is intended to be an intraluminal vascular prosthesis for use within a blood vessel, and the balloon catheter assembly is of the type commonly used in therapeutic coronary angioplasty procedures, it will be appreciated by those skilled in the art that modifications may be made to the present invention to allow the present invention to be used to load any type of prosthesis. The present invention is not just limited to stents that are designed to be deployed in the vasculature of a patient, but can be used to crimp any type of graft, prosthesis, liner or similar structure onto a delivery device or other apparatus. Further, the stent may be intended for delivery not only into coronary arteries, but also into any other body lumen. Other modifications can be made to the present invention by those skilled in the art without departing from the scope thereof.

## Claims

1. A stent-crimping tool (10) to temporarily affix a stent (A) onto a catheter assembly (D), comprising:
a base portion (14) having an arm intermediate portion (16) and pivoting portions (18,20) pivotally attached to the intermediate portion, and
a loop portion (12) attached to the pivoting arm portions (18,20), for use in supporting a portion of the catheter assembly (D) on which the stent is to be loaded, the loop portion (12) having a compressible and generally cylindrical opening (50) which is substantially uniformly compressible radially inwardly, upon the application of force to the said pivoting arm portions, to substantially uniformly and tightly crimp the stent onto the catheter assembly.

2. The stent-crimping tool (10) of claim 1, wherein the loop portion (12) comprises a sheet of flexible material (44) having first and second end portions (42,46), the first end portion comprising a plurality of elongate straps (40) extending from the first end portion of the sheet to terminal end regions (52), the sheet (44) having apertures (48) formed therethrough, wherein the elongate straps are threaded through the apertures formed in the sheet to establish the generally cylindrical opening (50), the opening being adapted to constrict when the elongate straps (40) and the second end (46) of the sheet are pulled in opposite directions.

3. The stent-crimping tool of claim 2, further comprising a pair of clips (38) for affixing the terminal end regions (52) of the elongate straps (40) and a portion of the second end (46) of the sheet (44) of the loop portion (12) to the pivoting arm portions, with the generally cylindrical opening (50) riding on the intermediate portion (16).

4. The stent-crimping tool (10) of claim 3, wherein each pivoting arm portion (18,20) has an engagement means (32)formed thereon and wherein the clips (38) have complementary engagement portions (54) adapted for engagement with the pivoting arm engagement means to retain the terminal end portions (52) and the second end (46) of the sheet (44) of the loop portion (12) that are positioned thereon.

5. The stent-crimping tool (10) of claim 4, wherein the sheet of flexible material (44) further has a top surface (22) and each pivoting arm portion (18, 20) further has a plurality of side edges (36) each side edge having a recess (34) disposed therein, and the engagement means (32) comprises a slot formed in the top surface for each pivoting arm portion, and the complimentary engagement portions (54) of the clips (38) each have a bar positioned on bottom surface (56) thereof adapted to fit into a corresponding slot (32), and a plurality of clip ends (58) adapted to fit into the recesses (34) in the side edges (36) of the pivoting arm portions (18,20).

6. The stent-crimping tool (10) of claim 1, further comprising a protective layer (60) which covers the loop portion (12) and which prevents the pivoting arm portions (18, 20) from being prematurely pivoted downwardly from the intermediate portion.

7. The stent-crimping tool (10) of claim 1, wherein the base portion (14) is formed from a single block of generally rigid material, wherein the pivoting arm portions (18, 20) and the intermediate portions (16) are formed by longitudinal slots (26, 28) formed in the rigid material extending from a top surface (22) to near a bottom surface (24) of the single block of material, leaving unslotted areas of material defining a pair of hinges (30A, 30B), so that in use, the pivoting arm portions can be pivoted on the pair of hinges.

8. A method of substantially uniformly and tightly crimping an intravascular stent (A) onto a catheter assembly (D), comprising the steps of:
providing a device (10) comprising a base portion (14) having an intermediate portion (16) and pivoting arm portions (18, 20) attached to the intermediate portion, and a loop portion (12) for use in supporting a portion of the catheter assembly (D) on which the stent (A) may be positioned, the loop portion having a compressible and generally cylindrical opening (50) which is substantially uniformly compressible radially inwardly upon the application of force thereto to substantially uniformly and tightly crimp the stent onto the catheter portion, the loop portion having end portions (42, 46) attached to the pivoting arm portions;
placing a portion of the catheter assembly, on which the stent is positioned, within the loop portion;
pivotally moving the pivoting arm portions relative to the intermediate portion to move the end portions of the loop portion in opposite directions thereby reducing the diameter of the generally cylindrical opening to apply compressive force to compress the stent radially inwardly, to substantially uniformly and tightly crimp the stent onto the catheter portion; and
releasing the compressive force to enable radially outward expansion of the generally cylindrical opening to enable the stent and catheter assembly to be withdrawn.

9. The method of claim 8, wherein the loop portion (12) comprises a sheet of flexible material (44), having first and second end (42, 46), the first end portion having a plurality of elongate straps (40) extending from the first end portion of the sheet to terminal end regions (52), the sheet having apertures (48) formed therethrough, wherein the elongate straps are threaded through the apertures formed in the sheet to establish the generally cylindrical loop opening, which loop opening is configured to constrict when the elongate straps and the second end (44) of the sheet are pulled in opposite directions.

10. The method of claim 8, wherein the device is covered with a removable protective layer (60) to protect the loop portion (12) and to prevent the pivoting arm portions (18, 20) from prematurely pivoting from the intermediate portion.

## Patentansprüche

1. Stent-Krimpwerkzeug (10) zum vorübergehenden Befestigen eines Stents (A) auf einer Katheteranordnung (D), umfassend:
einen Basisabschnitt (14) mit einem Mittelabschnitt (16) und Schwenkarmabschnitten (18, 20), die an dem Mittelabschnitt schwenkbar angebracht sind; und
einen Schleifenabschnitt (12), der an den Schwenkarmabschnitten (18, 20) angebracht ist, um bei Gebrauch einen Abschnitt der Katheteranordnung (D) zu halten, auf dem der Stent angebracht werden soll, wobei der Schleifenabschnitt (12) eine komprimierbare und allgemein zylindrische Öffnung (50) aufweist, die beim Ausüben von Kraft auf die Schwenkarmabschnitte im Wesentlichen gleichmäßig radial einwärts komprimierbar ist, um den Stent im Wesentlichen gleichmäßig und dicht auf die Katheteranordnung zu krimpen.

2. Stent-Krimpwerkzeug (10) nach Anspruch 1, wobei der Schleifenabschnitt (12) ein Blatt aus flexiblem Material (44) mit einem ersten und einem zweiten Endabschnitt (42, 46) aufweist, wobei der erste Endabschnitt eine Mehrzahl langgestreckter Streifen (40) aufweist, die von dem ersten Endabschnitt des Blatts zu Außenendbereichen (52) abstehen, wobei in dem Blatt (44) Öffnungen (48) ausgebildet sind, wobei die langgestreckten Streifen durch die in dem Blatt gebildeten Öffnungen hindurchgezogen werden, um die allgemein zylindrische Öffnung (50) zu bilden, wobei die Öffnung so ausgebildet ist, dass sie sich verengt, wenn die langgestreckten Streifen (40) und das zweite Ende (46) des Blatts in entgegengesetzte Richtungen gezogen werden.

3. Stent-Krimpwerkzeug nach Anspruch 2, das ferner ein Paar von Klemmen (38) aufweist, um die Außenendbereiche (52) der langgestreckten Streifen (40) und einen Abschnitt des zweiten Endes (46) des Blatts (44) des Schleifenabschnitts (12) an den Schwenkarmabschnitten zu befestigen, wobei die allgemein zylindrische Öffnung (50) auf dem Mittelabschnitt (16) aufliegt.

4. Stent-Krimpwerkzeug (10) nach Anspruch 3, wobei an jedem Schwenkarmabschnitt (18, 20) ein Eingriffsmittel (32) ausgebildet ist, und wobei die Klemmen (38) komplementäre Eingriffsabschnitte (54) aufweisen, die zum Eingriff mit den Schwenkarmeingriffsmitteln dienen, um Außenendabschnitte (52) und das zweite Ende (46) des Blatts (44) des Schleifenabschnitts (12), die daran positioniert sind, zu halten.

5. Stent-Krimpwerkzeug (10) nach Anspruch 4, wobei das Blatt aus flexiblem Material (44) ferner eine Oberseite (22) aufweist und jeder Schwenkarmabschnitt (18, 20) ferner eine Mehrzahl von Seitenrändern (36) aufweist, wobei in jedem Seitenrand eine Vertiefung (34) angeordnet ist, und wobei das Eingriffsmittel (32) einen in der Oberseite jedes Schwenkarmabschnitts ausgebildeten Schlitz aufweist, und wobei die komplementären Eingriffsabschnitte (54) der Klemmen (38) jeweils eine an ihrer Unterseite (56) angeordnete Leiste aufweisen, die dazu ausgelegt ist, in einen entsprechenden Schlitz (32) zu passen, und wobei eine Mehrzahl von Klemmenenden (58) dazu ausgelegt ist, in die Vertiefungen (34) in den Seitenrändern (36) der Schwenkarmabschnitte (18, 20) zu passen.

6. Stent-Krimpwerkzeug (10) nach Anspruch 1, das ferner eine Schutzschicht (60) aufweist, die den Schleifenabschnitt (12) abdeckt und die verhindert, dass die Schwenkarmabschnitte (18, 20) vorzeitig von dem Mittelabschnitt nach unten verschwenkt werden.

7. Stent-Krimpwerkzeug (10) nach Anspruch 1, wobei der Basisabschnitt (14) aus einem Einzelblock aus allgemein starrem Material gebildet ist, wobei die Schwenkarmabschnitte (18, 20) und der Mittelabschnitt (16) durch in dem starren Material gebildete längliche Schlitze (26, 28) gebildet sind, die sich von einer Oberseite (22) zu nahe einer Unterseite (24) des einzelnen Materialblocks erstrecken, wobei sie ungeschlitzte Materialbereiche belassen, die ein Paar von Gelenken (30A, 30B) bilden, so dass bei Gebrauch die Schwenkarmabschnitte an dem Gelenkpaar verschwenkt werden können.

8. Verfahren zum im Wesentlichen gleichmäßigen und dichten Krimpen eines intravaskularen Stents (A) auf eine Katheteranordnung (D), welches die Schritte aufweist:
Vorsehen einer Vorrichtung (10), die einen Basisabschnitt (14) mit einem Mittelabschnitt (16) und an dem Mittelabschnitt angebrachten Schwenkarmabschnitten (18, 20) sowie einen Schleifenabschnitt (12) zum Halten eines Abschnitts der Katheteranordnung (D), an dem der Stent (A) positioniert werden kann, aufweist, wobei der Schleifenabschnitt eine komprimierbare und allgemein zylindrische Öffnung (50) aufweist, die beim Ausüben von Kraft auf diese im Wesentlichen gleichmäßig radial einwärts komprimierbar ist, um den Stent im Wesentlichen gleichmäßig und dicht auf den Katheterabschnitt zu krimpen, wobei der Schleifenabschnitt an den Schwenkarmabschnitten angebrachte Endabschnitte (42, 46) aufweist;
Anordnen eines Abschnitts der Katheteranordnung, an der der Stent positioniert ist, in dem Schleifenabschnitt;
Verschwenken der Schwenkarmabschnitte relativ zu dem Mittelabschnitt, um die Endabschnitte des Schleifenabschnitts in entgegengesetzte Richtungen zu bewegen, um hierdurch den Durchmesser der allgemein zylindrischen Öffnung zu reduzieren und Kompressionskräfte auszuüben, um den Stent radial einwärts zu komprimieren, um den Stent im Wesentlichen gleichmäßig und dicht auf den Katheterabschnitt zu krimpen; und
Lösen der Kompressionskraft, um eine Erweiterung der allgemein zylindrischen Öffnung nach radial aussen zu ermöglichen, damit die Stent-und-Katheter-Anordnung entfernt werden kann.

9. Verfahren nach Anspruch 8, wobei der Schleifenabschnitt (12) ein Blatt aus einem flexiblen Material (44) mit einem ersten und einem zweiten Ende (42, 46) aufweist, wobei der erste Endabschnitt eine Mehrzahl langgestreckter Streifen (40) aufweist, die von dem ersten Endabschnitt des Blatts zu Außenendbereichen (42) abstehen, wobei das Blatt von Öffnungen (48) durchsetzt ist, wobei die langgestreckten Streifen durch die in dem Blatt ausgebildeten Öffnungen hindurchgezogen werden, um die allgemein zylindrische Schleifenöffnung zu bilden, wobei die Schleifenöffnung konfiguriert ist, um sich zu verengen, wenn die langgestreckten Streifen und das zweite Ende (44) des Blatts in entgegengesetzte Richtungen gezogen werden.

10. Verfahren nach Anspruch 8, wobei die Vorrichtung mit einer entfernbaren Schutzschicht (60) abgedeckt ist, um den Schleifenabschnitt (12) zu schützen und um zu verhindern, dass die Schwenkarmabschnitte (28) vorzeitig von dem Mittelabschnitt verschwenkt werden.

## Revendications

1. Outil de serrage de stent (10) pour fixer provisoirement un stent (A) sur un ensemble formant cathéter (D), comprenant :
une partie de base (14) ayant une partie intermédiaire (16) et des parties de bras pivotants (18, 20) fixées de manière pivotante sur la partie intermédiaire ; et
une partie en boucle (12) fixée sur les parties de bras pivotants (18, 20), destinée à être utilisée afin de supporter une partie de l'ensemble formant cathéter (D) sur lequel doit être chargé le stent, la partie en boucle (12) ayant une ouverture (50) compressible et sensiblement cylindrique qui peut être comprimée de façon uniforme radialement vers l'intérieur, lorsqu'on applique une force sur lesdites parties de bras pivotants, afin de comprimer sensiblement uniformément et de manière serrée le stent sur l'ensemble formant cathéter.

2. Outil de serrage de stent (10) selon la revendication 1, dans lequel la partie en boucle (12) comprend une feuille de matériau flexible (44) ayant des première et seconde parties d'extrémité (42, 46), la première partie d'extrémité comprenant une pluralité de bandelettes allongées (40) s'étendant depuis la première partie d'extrémité de la feuille jusqu'aux régions d'extrémité terminales (52), la feuille (44) ayant des trous (48) formés à travers elle, dans lesquels les bandelettes allongées sont insérées de manière à former l'ouverture sensiblement cylindrique (50), celle-ci étant susceptible de se resserrer lorsque les bandelettes allongées (40) et la seconde extrémité (46) de la feuille sont tirées dans des directions opposées.

3. Outil de serrage de stent selon la revendication 2, comprenant en outre une paire de pinces (38) destinées à fixer les régions d'extrémité terminales (52) des bandelettes allongées (40) et une partie de la seconde extrémité (46) de la feuille (44) de la partie en boucle (12) sur les parties de bras pivotants, l'ouverture sensiblement cylindrique (50) passant sur la partie intermédiaire (16).

4. Outil de serrage de stent (10) selon la revendication 3, dans lequel chaque partie de bras pivotant (18, 20) présente des moyens de prise (32) et dans lequel les pinces (38) présentent des parties de prise complémentaires (54) adaptées pour venir en prise avec les moyens de prise des bras pivotants afin de retenir les parties d'extrémité terminales (52) et la seconde extrémité (46) de la feuille (44) de la partie en boucle (12).

5. Outil de serrage de stent (10) selon la revendication 4, dans lequel la partie de base (14) présente en outre une surface supérieure (22), chaque partie de bras pivotant (18, 20) présente en outre une pluralité de bords latéraux (36), chaque bord latéral comporte un creux (34), les moyens de prise (32) sont constitués par une rainure ménagée dans la surface supérieure de chaque partie de bras pivotant, les parties de prise complémentaires (54) des pinces (38) présentent chacune une nervure placée sur leur surface inférieure (56) et susceptible de s'insérer dans la rainure correspondante (32), et une pluralité d'extrémités de pince (58) susceptibles de s'insérer dans les creux (34) ménagés dans les bords latéraux (36) des parties de bras pivotants (18, 20).

6. Outil de serrage de stent (10) selon la revendication 1, comprenant en outre une couche protectrice (60) qui recouvre la partie en boucle (12) et qui empêche les parties de bras pivotants (18, 20) d'être pivotées prématurément vers le bas par rapport à la partie intermédiaire.

7. Outil de serrage de stent (10) selon la revendication 1, dans lequel la partie de base (14) est formée à partir d'un seul bloc de matériau généralement rigide, les parties de bras pivotants (18, 20) et la partie intermédiaire (16) sont formées en pratiquant dans le matériau rigide des fentes longitudinales (26, 28) s'étendant depuis la surface supérieure (22) pour s'approcher d'une surface inférieure (24) du bloc de matériau unique, en laissant des zones de matériau non fendues pour définir une paire d'articulations (30A, 30B), de telle sorte qu'en utilisation, les parties de bras pivotants puissent être pivotées autour de la paire d'articulations.

8. Procédé pour comprimer sensiblement uniformément et de manière serrée un stent intravasculaire (A) sur un ensemble formant cathéter (D), comprenant les étapes consistant à :
prévoir un dispositif (10) comprenant une partie de base (14) ayant une partie intermédiaire (16) et des parties de bras pivotants (18, 20) fixées sur la partie intermédiaire, et une partie en boucle (12) destinée à être utilisée pour supporter une partie de l'ensemble formant cathéter (D) sur laquelle peut être placé le stent (A), la partie en boucle ayant une ouverture (50) compressible et sensiblement cylindrique qui est compressible de façon uniforme radialement vers l'intérieur lorsqu'on lui applique une force afin de comprimer sensiblement uniformément et de manière serrée le stent sur la partie de cathéter, la partie en boucle ayant des parties d'extrémité (42, 46) fixées sur les parties de bras pivotants ;
placer une partie de l'ensemble formant cathéter, sur lequel est placé le stent, à l'intérieur de la partie en boucle ;
déplacer de manière pivotante les parties de bras pivotants par rapport à la partie intermédiaire pour déplacer les parties d'extrémité de la partie en boucle dans des directions opposées, réduisant ainsi le diamètre de l'ouverture sensiblement cylindrique afin d'appliquer une force de compression qui comprime le stent radialement vers l'intérieur, et de comprimer sensiblement uniformément et de manière serrée le stent sur la partie de cathéter ; et
relâcher la force de compression pour permettre la dilatation radialement vers l'extérieur de l'ouverture sensiblement cylindrique et permettre le retrait du stent et de l'ensemble formant cathéter.

9. Procédé selon la revendication 8, dans lequel la partie en boucle (12) comprend une feuille de matériau flexible (44) ayant des première et seconde extrémités (42, 46), la première partie d'extrémité ayant une pluralité de bandelettes allongées (40) s'étendant de la première partie d'extrémité de la feuille vers les régions d'extrémité terminales (52), la feuille comportant des trous (48) ménagés à travers celle-ci, dans lesquels les bandelettes allongées sont insérées pour former l'ouverture de boucle sensiblement cylindrique, laquelle ouverture de boucle est configurée pour se resserrer lorsque les bandelettes allongées et la seconde extrémité (44) de la feuille sont tirées dans des directions opposées.

10. Procédé selon la revendication 8, dans lequel le dispositif est recouvert d'une couche protectrice amovible (60) pour protéger la partie en boucle (12) et pour empêcher que les parties de bras pivotants (18, 20) ne pivotent prématurément par rapport à la partie intermédiaire.
